Europäisches Patentamt

European Patent Office

Office européen des brevets

(11) Publication number: **0 441 347 B1**

# EUROPEAN PATENT SPECIFICATION

(45) Date of publication of patent specification: **31.08.94** (51) Int. Cl.⁵: **C07C 63/38**, C07C 51/493

(21) Application number: **91101613.7**

(22) Date of filing: **06.02.91**

(54) **Method of producing naphthalene dicarboxylic acid.**

(30) Priority: **08.02.90 JP 27095/90**
**18.12.90 JP 411285/90**

(43) Date of publication of application:
**14.08.91 Bulletin 91/33**

(45) Publication of the grant of the patent:
**31.08.94 Bulletin 94/35**

(84) Designated Contracting States:
**DE FR GB**

(56) References cited:
**US-A- 3 650 907**

**CHEMICAL ABSTRACTS, vol. 102, no. 24, May 27, 1985, Columbus, Ohio, USA W. XUet al. "Purification and identification of 2,6- naphthalenedicarboxylic acid"page 2, abstract-No. 185 547z**

(73) Proprietor: **KAWASAKI STEEL CORPORATION**
**1-28, Kitahonmachi-dori 1-Chome**
**Chuo-Ku, Kobe-Shi Hyogo-Ken (JP)**

(72) Inventor: **Yoshino, Kenji, c/o Kawasaki Steel Corporation**
**Technical Research Div.,**
**1, Kawasaki-Cho**
**Chiba City, Chiba Pref. (JP)**
Inventor: **Wakui, Tadahiro, c/o Kawasaki Steel Corporation**
**Technical Research Div.,**
**1, Kawasaki-Cho**
**Chiba City, Chiba Pref. (JP)**

(74) Representative: **Henkel, Feiler, Hänzel & Partner**
**Möhlstrasse 37**
**D-81675 München (DE)**

## Description

This invention relates to a method of producing a high purity naphthalene dicarboxylic acid.

Naphthalene dicarboxylic acid (hereinafter abbreviated as NDCA), particularly 2,6-naphthalene dicarboxylic acid (2,6-NDCA) is noticed as a starting material for dye and medicine and a starting material for polyethylene naphthoate resin (PEN) and liquid crystal polyester resin (LCP). In case of PEN using dimethyl 2,6-naphthalene dicarboxylate (2,6-NDCA dimethyl) as a starting material, since 2,6-NDCA dimethyl is easy in the purification and available in a high purity, the production has already been put into practical use. However, in case of LCP using 2,6-NDCA as a starting material, excellent properties are attained, but it is difficult to obtain a high purity 2,6-NDCA, so that the production is not yet put into practical use.

The reason why the obtainment of high purity 2,6-NDCA is difficult is due to the fact that the purification is very difficult as mentioned in the following features 1) - 3):

1) There is substantially no solvent sufficiently dissolving 2,6-NDCA, so that the recrystallization is impossible.

2) The vapor pressure of 2,6-NDCA is very low, so that the purification through distillation or sublimation is impossible.

3) Since 2,6-NDCA does not melt (which has no melting point and decomposes by heating), the crystallization is impossible.

Up to the present, many methods of purifying 2,6-NDCA have been proposed, but they are not for direct purification. For example, there have been known a method wherein 2,6-NDCA is converted into an acid chloride and then purified (Japanese Patent laid open No. 62-169747), a method wherein 2,6-NDCA is converted into an alkali salt and then purified (Japanese Patent Application Publications No. 45-7738, No. 45-13096, No. 52-20993, No. 52-20994, No. 56-3858 and No. 57-36901), a method wherein 2,6-NDCA, obtained by thermal rearrangement of di-K 1,8-naphthalenedicarboxylate, is purified by esterification and hydrolysis (Chemical Abstracts Vol. 102 No.24, May 27 1985, abstract No. 185547z, Xu, W. et al.), , a method wherein 2,6-NDCA is converted into an amine salt and then purified (Japanese Patent Application Publications No. 56-48498 and No. 57-14331), a method wherein 2,6-NDCA is converted into an ammonium salt and then purified (Japanese Patent laid open No. 51-52163).

In the method of purifying 2,6-NDCA after the conversion into acid chloride, however, it is required to use an expensive halogenating agent such as thionyl chloride. Furthermore, this method can not be said to be an industrially suitable one considering the treatment for chloride by-produced in the turning of acid chloride to carboxylic acid and the material of the equipment durable to corrosion. On the other hand, in the methods of purifying after the conversion into various salts, it is difficult to remove position isomers and monocarboxylic acid included as impurities in 2,6-NDCA, and a high purity usable as a starting material for LCP cannot be obtained.

A simple method of producing a high purity 2,6-NDCA usable as a starting material for LCP is the production from 2,6-NDCA dialkylester. Since 2,6-NDCA dialkylester, for example, 2,6-NDCA dimethyl can be purified by distillation or recrystallization, crude 2,6-NDCA is converted into dialkylester with an alcohol or the like and purified to obtain a high purity 2,6-NDCA dialkylester, from which a high purity 2,6-NDCA can be obtained.

However, when 2,6-NDCA dialkylester is converted into 2,6-NDCA through hydrolysis with a strong alkali, if the strong alkali is sodium hydroxide, the resulting 2,6-NDCA disodium salt is hardly soluble in water, so that the reaction contents change into a gel as the hydrolysis reaction proceeds and finally renders into a solid soap to stop the reaction. Even when using potassium hydroxide as a strong alkali, as the reaction proceeds, the reaction contents change into a slurry having a high viscosity, so that the reaction should be carried out with a fairly diluted solution for preventing such a change. Furthermore, the resulting 2,6-NDCA consumes the alkali to produce a salt, so that it is necessary to use an alkali in an amount of not less than equivalent of resulting carboxyl group. Moreover, the particle size of 2,6-NDCA slurry produced when the resulting 2,6-NDCA dialkali salt is neutralized with an acid is very small, so that the slurry has a high viscosity and a high water content. As a result, the treatment for solid-liquid separation is charged with difficulties and also the washing becomes difficult, so that there are caused problems that the alkali metal salt by-produced in the neutralization incorporates into final 2,6-NDCA and a great energy is taken for the drying after the washing.

On the other hand, the use of the acid catalyst can substantially solve the aforementioned drawbacks caused in the hydrolysis with the alkali catalyst, but the reaction is very slow and can not put into practical use. For instance, even when 2,6-NDCA is boiled in an aqueous solution of strong acid, no hydrolysis reaction proceeds.

It is, therefore, an object of the invention to solve the above problems of the conventional technique and to provide a method of easily and rapidly producing a high purity NDCA from NDCA dialkylester.

The inventors have made various studies with respect to a method of producing NDCA from NDCA dialkylester without an alkali catalyst and found that when a monocarboxylic acid containing no unsaturated bond group and having a carbon number of 1-10 is used as a solvent, an ester exchange reaction is easily caused by heating in the presence of an esterification catalyst to produce NDCA. Furthermore, it has been found that the resulting NDCA is insoluble in the above monocarboxylic acid solvent having a carbon number of 1-10 and is large in the particle size, so that the solid-liquid separation can easily be carried out and the high purity NDCA is obtained only by washing.

The invention is based on the above knowledge, and is characterized in that NDCA dialkylester is reacted within a temperature range of 70-350°C in a solvent inclusive of a monocarboxylic acid having a carbon number of 1-10 and selected from the group consisting of aliphatic, alicyclic and aromatic acids and mixtures thereof, wherein the aliphatic and alicyclic acids contain no multiple carbon-carbon bond and containing water in an amount of not more than 50% by weight in the presence of at least one esterification catalyst selected from the group consisting of strong acids, metal salts and metal oxides and then subjected to a solid-liquid separation to obtain a high purity naphthalene dicarboxylic acid.

The invention will concretely be described below.

According to the invention, NDCA dialkylester used as a starting material is made from NDCA (and its derivative) and aliphatic alcohol (and its derivative). As the NDCA dialkylester, there are 10 kinds of position isomers, but anyone of these isomers may be used. As to alkyl group of the NDCA dialkylester, the carbon number and branched degree are not particularly critical. The alkyl group preferably includes methyl group, ethyl group, propyl group, isopropyl group, butyl group, isobutyl group, hexyl group and octyl group, among which methyl group is favorable. As the NDCA dialkylester, mention may be made of dimethyl ester, diethyl ester, dipropyl ester and dioctyl ester of 1,2-, 1,3-, 1,4-, 1,5-, 1,6-, 1,7-, 1,8-, 2,3-, 2,6- and 2,7-naphthalene dicarboxylic acids and so on. Among these esters, 1,5-NDCA dimethyl, 2,6-NDCA dimethyl and 2,7-NDCA dimethyl are preferable from a view point of easiness in the conversion from NDCA to dialkylester and the value of resulting NDCA as an industrial material, and particularly 2,6-NDCA dimethyl is favorable.

The esterification catalyst used in the invention is a catalyst usually used for esterification reaction of carboxylic acid and alcohol or phenol. It includes strong acids, metal salts and metal oxides.

As the strong acid, mention may be made of sulfuric acid, nitric acid, hydrochloric acid, toluene sulfonic acid, fluoroacetic acid, boron trifluoride, strong acid-type ion exchange resin and Nafion® (trade name; made by E. I. Dupont), among which sulfuric acid is most preferable. As the metal salt and metal oxide, mention may be made of oxides of zinc, lead, copper, manganese, cobalt, iron, nickel, antimony, cadmium, tin, mercury, aluminum, bismuth, selenium and tellurium as well as sulfates, chlorides, sulfides, bromides, acetates and the like of these metals.

Among these esterification catalysts, the strong acid is suitable from a viewpoint that it is not preferable to incorporate metal ion into the resulting NDCA as an impurity. The most preferable strong acid is sulfuric acid because it is non-volatile and easily available.

As the amount of the catalyst becomes larger, the reaction is fast and NDCA is obtained in a high yield for a short time, but the treatment of waste liquid and the purification for removing the catalyst from the resulting NDCA come into problems. For example, when the strong acid is used as an esterification catalyst, the amount of the strong acid is 0.01-1.0 mol/kg, particularly 0.1-0.6 mol/kg as a concentration of hydrogen ion per 1 kg of the monocarboxylic acid solvent.

The term "monocarboxylic acid having a carbon number of 1-10 and selected from the group consisting of aliphatic, alicyclic and aromatic acids and mixtures thereof; wherein the aliphatic and alicyclic acids contain no multiple carbon-carbon bond" used in the invention means aliphatic, alicyclic and aromatic monocarboxylic acids having a carbon number of 1-10. In case of using unsaturated carboxylic acid, polyvalent carboxylic acid, aliphatic monocarboxylic acid having a carbon number of not less than 11 and alicyclic monocarboxylic acid having a carbon number of not less than 11 as a solvent, these acids themselves react by heating in the presence of the strong acid and hence the reaction product is incorporated into the resulting NDCA as an impurity. For example, in case of the unsaturated carboxylic acid, the reaction of the unsaturated bond is generated to cause gelation of the solvent and the coloration of the product. In case of the polyvalent carboxylic acid or the aliphatic or alycyclic monocarboxylic acid having a carbon number of not less than 11, black and insoluble product is created. When using the carboxylic acid having a carbon number of not less than 11 as a solvent irrespective of saturated, unsaturated, aromatic, aliphatic and alycyclic, the reaction rate lowers and the viscosity rises and the solid-liquid separation becomes difficult.

That is, the use of unsaturated carboxylic acid, polyvalent carboxylic acid and aliphatic and alicyclic monocarboxylic acids having a carbon number of not less than 11 can not achieve such an object of the invention that the high purity NDCA is obtained only by a simple washing purification after the solid-liquid separation of the reaction product.

Moreover, the monocarboxylic acids having a carbon number of 1-10 according to the invention may contain a relatively inactive group such as halogen atom, ether group or carbonyl group, and may be used alone or in admixture.

As the aliphatic monocarboxylic acid, mention may be made of formic acid, acetic acid, propionic acid, butyric acid, valeric acid, caproic acid, enanthic acid, caprylic acid, pelargonic acid and capric acid. As the alicyclic monocarboxylic acid, mention may be made of cyclopropane carboxylic acid, cyclobutane carboxylic acid, cyclopentane carboxylic acid, cyclohexane carboxylic acid and cycloheptane carboxylic acid. As the aromatic monocarboxylic acid, mention may be made of benzoic acid and parachloro benzoic acid. Moreover, ketone acid such as pyruvic acid, trifluoro acetic acid and phenyl acetic acid can be used. The monocarboxylic acid having a carbon number of 1-10 according to the invention is not limited to the aforementioned acids. As the monocarboxylic acid, formic acid, acetic acid, propionic acid and benzoic acid are preferable, among which acetic acid is most favorable.

According to the invention, the solvent contains water in addition to the monocarboxylic acid having a carbon number of 1-10. The water content is not more than 50% by weight. If the solvent does not contain water, an alkyl ester of monocarboxylic acid is produced in the reaction system by ester exchange reaction (acidolysis) between NDCA dialkylester and the monocarboxylic acid solvent. Since this reaction is equilibrium, in order to obtain the high purity NDCA after the completion of the reaction, it is required to use an equipment for removing the resulting alkyl ester of monocarboxylic acid from the reaction system.

On the other hand, when the solvent contains water, the resulting alkyl ester of monocarboxylic acid is further hydrolyzed with water into monocarboxylic acid and alcohol, so that the equilibrium is shifted to complete the reaction. Therefore, the amount of water in the solvent is preferable to be not less than 2 times of mol equivalent of NDCA dialkylester used as a starting material. However, if the water content in the solvent exceeds 50% by weight, the reaction is undesirably slow. The preferable water content in the solvent is not more than 20% by weight and not less than 2 times of mol equivalent of NDCA dialkylester as a starting material.

The amount of monocarboxylic acid solvent containing not more than 50% by weight of water to be used is not particularly restricted. If the amount of the solvent used is too large to NDCA, the yield per one batch decreases, which comes into problem in the production efficiency, while when it is too small, the reaction system forms a high viscosity slurry.

The preferable amount of the monocarboxylic acid solvent used is within a range of 100-1000 parts by weight based on 100 parts by weight of NDCA dialkylester.

Moreover, an inert solvent giving no influence on the reaction may be existent in the carboxylic acid solvent used in the invention. As such a solvent, there are typified, for example, hydrocarbons such as toluene, xylene, biphenyl, alkylnaphthalene, mineral oil and the like; methyl ethyl ketone, diphenyl ether and silicone oil.

According to the invention, the reaction temperature is required to be not lower than 70°C. When the reaction temperature is lower than 70°C, the reaction hardly proceeds. When the temperature exceeds 350°C, decarbonation and decomposition reaction of the resulting NDCA undesirably occurs. The preferable reaction temperature is within a range of 120-300°C.

The pressure in the reaction system is not particularly restricted, but it is desirable that when using a low boiling point carboxylic acid such as formic acid or acetic acid, the reaction temperature is raised by pressurization. The preferable temperature range under pressurization is 120-200°C. For instance, in case of acetic acid, when the temperature exceeds 200°C, the vapor pressure reaches to about 10 $kg/cm^2$, so that the pressure resistance of the reaction vessel comes into problem.

Moreover, the optimum combination of conditions in the production method according to the invention is that 2,6-NDCA dimethyl is reacted at 120-200°C in an aqueous solution of 80-95% by weight of acetic acid in the presence of sulfuric acid catalyst.

In order to utilize the easiness of solid-liquid separation between resulting NDCA and carboxylic acid solvent and simplicity of purification as a feature of the invention at maximum, it is preferable that the reaction is completed so as not to leave 2,6-NDCA dialkylester. Even if the reaction is not completed, no solvent dissolving the resulting NDCA is substantially existent, so that the high purity NDCA can simply be obtained by increasing only an operation of washing unreacted NDCA dialkylester with a solvent.

As a typical example of solid-liquid separation treatment, there are filtration by suction, filtration under pressure, centrifugal filtration, filtration under vacuum, gravity filtration, supernatant filtration, squeezing filtration and the like, which can favorably be used.

The following examples are given in illustration of the invention and are not intended as limitations thereof.

At first, analyses adopted in the following examples will be described below.

1) Quantitative determination of NDCA

Three quantitative determinations of NDCA in reaction product, NDCA monoalkyl ester (intermediate) and NDCA dialkylester (starting material) were made by HPLC analysis [column: Inertsil ODS (made by Gasukuro Kogyo Inc.) 4.2 x 250 mm, column temperature: 40°C, flow rate: 0.85 ml/min, detector: 260 nm UV, mobile phase: mixture of $CH_3CN/THF/KH_2PO_4/H_3PO_4$/tetrabutyl ammonium bromide/water, sample: 5 $\mu\ell$], from which NDCA purity was determined. Moreover, substances other than the above three compounds were not detected in all examples and comparative examples.

2) Measurement of whiteness degree

The whiteness degree was measured by a color analysis system of U3210 model (made by Hitachi, Ltd.) when a white standard plate of aluminum oxide was 100.

Example 1

Into a glass autoclave of 1$\ell$ capacity were charged 200 g of 2,6-NDCA dimethylester, 370 g of glacial acetic acid, 30 g of water and 6.0 g of concentrated sulfuric acid, and then temperature was raised at 5°C/min with stirring. As the reaction temperature rises, 2,6-NDCA dimethyl were gradually dissolved and finally rendered into a uniform transparent solution at 130°C. Then, white precipitates begun to produce at about 160°C.

When the temperature reached to 170°C, the reaction was continued at this temperature for 8 hours. Thereafter, the reaction product was cooled and filtered by suction to separate the solvent therefrom. The separated solid product was subjected to HPLC analysis as it was, whereby the purity of NDCA was measured. Moreover, the solvent after the filtration contained the solid matter in an amount corresponding to only 0.004% by weight of the resulting solid product. After the resulting solid product was ashed in a muffle furnace, a content of alkali metal ion was measured by a spectral analysis to be zero.

The solid product was washed with 400 ml of hot ethanol three times to sufficiently remove the solvent and acid and dried under vacuum to obtain a product powder. Then, the whiteness degree of the powder was measured to obtain a result shown in Table 1.

Examples 2-8

The same procedure as in Example 1 was repeated except that formic acid, propionic acid, valeric acid, caproic acid, caprilic acid, capric acid or benzoic acid was used instead of glacial acetic acid as a solvent. In case of only capric acid, the reaction solution was colored into yellow, but in case of the other solvents, the reaction solution was not colored likewise Example 1.

In case of benzoic acid, the whole of the reaction system was solidified in the cooling after the completion of the reaction, so that it was homogeneously pulverized and then subjected to HPLC analysis. On the other hand, the measurement of whiteness degree was carried out by reheating the powder, subjecting to solid-liquid separation through hot filtration and removing the solvent through washing with hot ethanol. The measured results are also shown in Table 1.

Comparative Examples 1-3

The same procedure as in Example 1 was repeated except that lauric acid, palmitic acid or adipic acid was used instead of glacial acetic acid as a solvent. The color of the reaction solution was brown in case of lauric acid and black in case of palmitic acid and adipic acid.

In any case, the whole of the reaction system was solidified in the cooling after the completion of the reaction, so that the HPLC analysis and measurement of whiteness degree were made in the same manner as in Example 8. The results are also shown in Table 1.

## Table 1

|  | Carboxylic acid solvent (carbon number) | Purity of 2,6-NDCA (%) | Whiteness degree of 2,6-NDCA |
|---|---|---|---|
| Example 2 | formic acid (C1) | 99 | 93.4 |
| Example 1 | acetic acid (C2) | 99 | 93.6 |
| Example 3 | propionic acid (C3) | 99 | 93.2 |
| Example 4 | valeric acid (C5) | 92 | 92.5 |
| Example 5 | caproic acid (C6) | 86 | 92.8 |
| Example 6 | caprilic acid (C8) | 82 | 90.3 |
| Example 7 | capric acid (C10) | 71 | 90.2 |
| Example 8 | benzoic acid (C7) | 78 | 93.5 |
| Comparative Example 1 | lauric acid (C12) | 65 | 73.0 |
| Comparative Example 2 | palmitic acid (C16) | 58 | 28.0 |
| Comparative Example 3 | adipic acid (C6) | 55 | 15.0 |

Comparative Example 4

The same procedure as in Example 1 was repeated except that methacrylic acid was used instead of glacial acetic acid as a solvent. In this case, the gelation of the solvent caused in the course of temperature rising, and consequently the ester exchange reaction did not proceed.

Examples 9-11

The same procedure as in Example 1 was repeated except that 12.0 g of concentrated hydrochloric acid (36%), 6.0 g of stannous chloride or 6.0 g of zinc chloride was added instead of 6.0 g of concentrated sulfuric acid. The measured results are shown in Table 2.

Comparative Examples 5, 6

The same procedure as in Example 1 was repeated except that no acid was added or 4.0 g of phosphoric acid was added instead of 6.0 g of concentrated sulfuric acid. The results are also shown in Table 2.

Table 2

| | Catalyst | Purity of 2,6-NDCA (%) | Whiteness degree of 2,6-NDCA |
|---|---|---|---|
| Comparative Example 5 | none | 0 | - |
| Comparative Example 6 | phosphoric acid | 0.3 | 92.1 |
| Example 9 | hydrochloric acid | 99 | 93.0 |
| Example 10 | stannous chloride | 45 | 91.6 |
| Example 11 | zinc chloride | 37 | 92.0 |

Examples 12-18, Comparative Example 7

The same procedure as in Example 1 was repeated except that the reaction temperature was 50°C (Comparative Example 7), 80°C, 110°C, 130°C, 140°C, 150°C, 160°C or 200°C (Examples 12-18). The results are shown in Table 3.

Table 3

| | Reaction temperature (°C) | Purity of 2,6-NDCA (%) |
|---|---|---|
| Comparative Example 7 | 50 | 0 |
| Example 12 | 80 | 5 |
| Example 13 | 110 | 24 |
| Example 14 | 130 | 60 |
| Example 15 | 140 | 94 |
| Example 16 | 150 | 96 |
| Example 17 | 160 | 97 |
| Example 18 | 200 | 100 |

Examples 19-24

The same procedure as in Example 1 was repeated except that the amount of concentrated sulfuric acid added was 1 g, 2 g, 3 g, 4 g, 10 g or 15 g, and thereafter the contents (wt%) of 2,6-NDCA, 2,6-NDCA monomethyl and 2,6-NDCA dimethyl in the reaction product were measured by HPLC analysis. The results are shown in Table 4.

## EP 0 441 347 B1

<div align="center">Table 4</div>

| | Concentrated sulfuric acid (g) | Content (wt%) | | |
|---|---|---|---|---|
| | | 2,6-NDCA | 2,6-NDCA monomethyl | 2,6-NDCA dimethyl |
| Example 19 | 1 | 89.4 | 10.5 | 0.1 |
| Example 20 | 2 | 97.0 | 3.0 | 0 |
| Example 21 | 3 | 97.9 | 2.1 | 0 |
| Example 22 | 4 | 98.2 | 1.8 | 0 |
| Example 23 | 10 | 98.7 | 1.3 | 0 |
| Example 24 | 15 | 99.0 | 1.0 | 0 |

Examples 25-31, Comparative Examples 8, 9

In to the same reaction vessel as in Example 1 were charged 200 g of 2,6-NDCA dimethyl, 6.0 g of concentrated sulfuric acid and 400 g of an aqueous solution of acetic acid having a weight concentration of 100% (glacial acetic acid), 97.5%, 95%, 90%, 80%, 60%, 50% (Examples 25-31), 40% or 0% (water) (Comparative Examples 8, 9), which were reacted at 160°C for 8 hours. The reaction product was subjected to HPLC analysis in the same manner as in Example 1. The results are shown in Table 5.

<div align="center">Table 5</div>

| | Concentration of acetic acid (wt%) | Content (wt%) | | |
|---|---|---|---|---|
| | | 2,6-NDCA | 2,6-NDCA monomethyl | 2,6-NDCA dimethyl |
| Example 25 | 100 | 35.2 | 53.6 | 11.2 |
| Example 26 | 97.5 | 56.0 | 32.1 | 11.9 |
| Example 27 | 95 | 93.2 | 6.8 | 0 |
| Example 28 | 90 | 97.6 | 2.4 | 0 |
| Example 29 | 80 | 94.3 | 5.7 | 0 |
| Example 30 | 60 | 45.2 | 42.6 | 12.2 |
| Example 31 | 50 | 23.2 | 11.1 | 65.7 |
| Comparative Example 8 | 40 | 0.5 | 1.0 | 98.5 |
| Comparative Example 9 | 0 | 0 | 0 | 100 |

Examples 32, 33

The same procedure as in Example 1 was repeated except that 2,7-NDCA dimethyl or 1,5-NDCA dimethyl was used instead of 2,6-NDCA. In any case, the corresponding NDCA was substantially quantitatively obtained. The results are shown in Table 6.

## Table 6

|  | Starting material | Purity of corresponding NDCA (%) | Whiteness degree |
|---|---|---|---|
| Example 32 | 2,7-NDCA dimethyl | 99.2 | 95.0 |
| Example 33 | 1,5-NDCA dimethyl | 99.7 | 94.3 |

### Comparative Example 10

Into a reaction vessel were charged 100 g of 2,6-NDCA dimethyl, 60 g of potassium hydroxide and 120 g of water, which were reacted at 100°C for 10 hours with stirring. The reaction solution was a high viscosity slurry. To the reaction solution was added 600 g of water to completely dissolve the resulting slurry of 2,6-NDCA dipotassium salt, which was filtered to separate unreacted 2,6-NDCA dimethyl. Then, the filtrate was adjusted to pH = 3 with hydrochloric acid. At the time of pH adjustment, white solid of 2,6-NDCA precipitated in form of a slurry. The viscosity of this slurry was measured to be 6500 centipoises, so that the solid-liquid separation was impossible owing to the high viscosity. For this end, the viscosity of the slurry was decreased to 1000 centipoises by adding 600 g of water, whereby the solid-liquid separation was made by filtration under vacuum. Next, the washing and the solid-liquid separation were repeated three times with 1000 g in total of water to remove potassium chloride produced in the neutralization. Thereafter, 2,6-NDCA containing water after the washing was dehydrated by means of a centrifugal separating machine. The water content after the dehydration was 67% by weight. This product was dried to obtain 2,6-NDCA. The yield was 97%.

When the content of metal ion was examined in the same manner as in Example 1, it has been confirmed that 2,6-NDCA contained 5200 ppm of potassium ion.

### Comparative Example 11

Into a reaction vessel were charged 60 g of 2,6-NDCA dimethyl, 26 g of sodium hydroxide and 100 g of water, which were reacted at 100°C with stirring. With the lapse of time, the viscosity of the reaction solution rose and was completely solidified after 6 hours. As a result, the stirring was impossible, so that the reaction was stopped. In this case, the yield of 2,6-NDCA was 60%.

As mentioned above, according to the invention, the particular carboxylic acid is acted as a starting material for ester exchange reaction and a solvent in the presence of an esterification catalyst, whereby a high purity naphthalene dicarboxylic acid can easily be obtained from a dialkylester of naphthalene dicarboxylic acid.

## Claims

1. A method of producing naphthalene dicarboxylic acids, characterized in that
   a dialkylester of naphthalene dicarboxylic acid is reacted within a temperature range of 70-350°C in a solvent inclusive of a monocarboxylic acid having a carbon number of 1-10 and selected from the group consisting of aliphatic, alicyclic and aromatic acids and mixtures thereof, wherein the aliphatic and alicyclic acids contain no multiple carbon-carbon bond and containing water in an amount of not more than 50% by weight in the presence of at least one esterification catalyst selected from the group consisting of strong acids, metal salts and metal oxides and then subjected to a solid-liquid separation to obtain a high purity naphthalene dicarboxylic acid.

2. The method according to claim 1, wherein said solvent contains not more than 20% by weight of water.

3. The method according to claim 1 or 2, wherein said dialkylester of naphthalene dicarboxylic acid is selected from the group consisting of dimethyl 1,5-naphthalene dicarboxylate, dimethyl 2,6-naphthalene dicarboxylate and dimethyl 2,7-naphthalene dicarboxylate.

4. The method according to any of claims 1 to 3, wherein said monocarboxylic acid is at least one acid selected from the group consisting of formic acid, acetic acid, propionic acid and benzoic acid.

5. The method according to claim 4, wherein said monocarboxylic acid is acetic acid.

6. The method according to any of claims 1 to 5, wherein said strong acid is selected from the group consisting of sulfuric acid, nitric acid, hydrochloric acid, fluoroacetic acid, boron trifluoride, strong acid type ion exchange resin and Nafion®.

7. The method according to claim 6, wherein said strong acid is sulfuric acid.

8. The method according to any of claims 1 to 5, wherein a metal of said metal oxide and said metal salt is selected from the group consisting of tin, zinc, lead, copper, manganese, cobalt, iron, nickel, antimony, aluminum, bismuth, selenium, tellurium, cadmium and mercury.

9. The method according to claim 1, wherein said reaction temperature is 120-200°C.

**Patentansprüche**

1. Verfahren zur Herstellung von Naphthalindicarbonsäuren, dadurch gekennzeichnet, daß zur Gewinnung einer hochreinen Naphthalindicarbonsäure ein Dialkylester einer Naphthalindicarbonsäure innerhalb eines Temperaturbereichs von 70-350°C in einem Lösungsmittel mit einer Monocarbonsäure ohne Gruppe mit einer ungesättigten Bindung und mit einer Kohlenstoffzahl von 1 bis 10 sowie mit Wasser in einer Menge von nicht mehr als 50 Gew.-% in Gegenwart mindestens eines Veresterungskatalysators, ausgewählt aus der Gruppe starke Säuren, Metallsalze und Metalloxide, reagieren gelassen wird, worauf eine Feststoff/Flüssigkeits-Trennung durchgeführt wird.

2. Verfahren nach Anspruch 1, wobei das Lösungsmittel nicht mehr als 20 Gew.-% Wasser enthält.

3. Verfahren nach Anspruch 1 oder 2, wobei der Dialkylester einer Naphthalindicarbonsäure aus der Gruppe Dimethyl-1,5-naphthalindicarboxylat, Dimethyl-2,6-naphthalindicarboxylat und Dimethyl-2,7-naphthalindicarboxylat ausgewählt wird.

4. Verfahren nach einem der Ansprüche 1 bis 3, wobei die Monocarbonsäure aus mindestens einer Säure, ausgewählt aus der Gruppe Ameisensäure, Essigsäure, Propionsäure und Benzoesäure, besteht.

5. Verfahren nach Anspruch 4, wobei die Monocarbonsäure aus Essigsäure besteht.

6. Verfahren nach einem der Ansprüche 1 bis 5, wobei die starke Säure aus der Gruppe Schwefelsäure, Salpetersäure, Chlorwasserstoffsäure, Fluoressigsäure, Bortrifluorid, einem stark sauren Ionenaustauscherharz und Nafion$^R$ ausgewählt ist.

7. Verfahren nach Anspruch 6, wobei die starke Säure aus Schwefelsäure besteht.

8. Verfahren nach einem der Ansprüche 1 bis 5, wobei ein Metall des Metalloxids und Metallsalzes aus der Gruppe Zinn, Zink, Blei, Kupfer, Mangan, Kobalt, Eisen, Nickel, Antimon, Aluminium, Wismut, Selen, Tellur, Cadmium und Quecksilber ausgewählt ist.

9. Verfahren nach Anspruch 1, wobei die Reaktionstemperatur 120 bis 200°C beträgt.

**Revendications**

1. Un procédé de production d'acides naphtalène-dicarboxyliques, caractérisé en ce qu'un dialkylester d'acide naphtalène-dicarboxylique est mis en réaction dans une plage de température de 70-350°C dans un solvant comportant un acide monocarboxylique ne contenant aucun groupe de liaison non saturée et présentant un nombre d'atomes de carbone de 1-10 et contenant de l'eau suivant une quantité non supérieure à 50 % en poids en présence d'au moins un catalyseur d'estérification sélectionné dans le groupe constitué par les acides forts, les sels de métaux et les oxydes métalliques, puis soumis à une séparation solide-liquide pour obtenir un acide naphthalène-dicarboxylique de pureté élevée.

2. Le procédé selon la revendication 1, dans lequel ledit solvant ne contient pas plus de 20% en poids d'eau.

3. Le procédé selon la revendication 1 ou 2, dans lequel ledit dialkylester d'acide naphthalène-dicarboxylique est sélectionné dans le groupe constitué par le dicarboxylate de diméthyle 1,5-naphthalène, le dicarboxylate de diméthyle 2,6-naphthalène et le dicarboxylate de diméthyle 2,7-naphthalène.

4. Le procédé selon l'une quelconque des revendications 1 à 3, dans lequel ledit acide monocarboxylique est au moins un acide sélectionné dans le groupe constitué par l'acide formique, l'acide acétique, l'acide propionique et l'acide benzoïque.

5. Le procédé selon la revendication 4, dans lequel ledit acide monocarboxylique est l'acide acétique.

6. Le procédé selon l'une quelconque des revendications 1 à 5, dans lequel ledit acide fort est sélectionné dans le groupe constitué par l'acide sulfurique, l'acide nitrique, l'acide hydrochlorique, l'acide fluoroacétique, le trifluorure de bore, une résine d'échange d'ions du type acide fort et le Nafion®.

7. Le procédé selon la revendication 6, dans lequel ledit acide fort est l'acide sulfurique.

8. Le procédé selon l'une quelconque des revendications 1 à 5, dans lequel un métal dudit oxyde métallique et dudit sel métallique est sélectionné dans le groupe constitué par l'étain, le zinc, le plomb, le cuivre, le manganèse, le cobalt, le fer, le nickel, l'antimoine, l'aluminium, le bismuth, le sélénium, le tellure, le cadmium et le mercure.

9. Le procédé selon la revendication 1, dans lequel ladite température de réaction est de 120-200°C.